(19) Europäisches Patentamt
European Patent Office
Office européen des brevets

(11) Publication number: 0 349 902
A2

(12) EUROPEAN PATENT APPLICATION

(21) Application number: 89111854.9

(22) Date of filing: 29.06.89

(51) Int. Cl.[4]: C07D 213/803 , C07D 213/80 , A61K 31/455

Claims for the following Contracting States: ES + GR.

(30) Priority: 08.07.88 IT 2130088

(43) Date of publication of application: 10.01.90 Bulletin 90/02

(84) Designated Contracting States: AT BE CH DE ES FR GB GR IT LI LU NL SE

(71) Applicant: CHIESI FARMACEUTICI S.p.A.
Via Palermo, 26/A
I-43100 Parma(IT)

(72) Inventor: Chiesi, Paolo
Via Palermo, 26/A
I-43100 Parma(IT)
Inventor: Servadio, Vittorino
Via Palermo, 26/A
I-43100 Parma(IT)
Inventor: Pighi, Roberto
Via Palermo, 26/A
I-43100 Parma(IT)

(74) Representative: Minoja, Fabrizio
Studio Consulenza Brevettuale Via Rossini, 8
I-20122 Milano(IT)

# (54) Processes for the preparation of morniflumate and analogous compounds.

(57) Novel processes for the preparation of morniflumate and analogous compounds are described, which processes allow to obtain directly the product as the free base, according to simplified synthesis schemes and in significantly higher yields than those obtained by the prior art processes.

The processes of the present invention are particularly advantageous for industrial applications.

EP 0 349 902 A2

## PROCESSES FOR THE PREPARATION OF MORNIFLUMATE AND ANALOGOUS COMPOUNDS

Morniflumate is the niflumic acid β-morpholinoethyl ester and has been described for the firts time by C. Hoffmann in French Patent n° 7963M, filed on October 3, 1968. Niflumic acid is a compound of the phenamates class, known due to the analgesic, antiinflammatory and antipyretic activities thereof. Esterification of niflumic acid with β-morpholinethanol or other hydroxyalkylamino groups, such as those disclosed in FR pat. n° 7963M as well as in the addition certificate thereof, published with FR pat.n° 2 187 317 had, according the Authors, the following purposes:

- improving the drug tolerability, reducing the injuring effects on gastrointestinal mucosa common to all the antiinflammatory non steroidal drugs (AINS);
- increasing the liposolubility of the compound and accordingly the transmucosal absorption thereof, mainly in view of the preparation of rectal formulations.

However, such a second object has not been reached, as morniflumate in a rectal formulation would be absorbed, in man, in remarkably lower amounts than those reached by the oral formulations (Backer P. et al, Acta Ther. 9(4), 333-343, 1983).

The Applicant, as a consequence of extensive original studies on this molecule, proved that morniflumate, when administered by the oral route, not only causes no damages on gastrointestinal mucosa, but even exerts a dose-related gastro-protective action on gastric necrotic lesions induces by absolute ethanol and other agents, and also inhibits hemorrhagic gastric lesions induced by such widely therapeutically used AINS as acetylsalicylic acid, indomethacin, diclofenac, ketoprofen, naproxen, phenylbutazone and by niflumic acid itself (Schiantarelli P. et al: Agents Actions 14(2), 247-255, 1984, and Arzneim. Forsch. 34(II) 8, 885-890, 1984).

The results from these studies are the object of a family patent and precisely: IT 1 150 194, GB 2 17 238, BE 895 976, FR 8 302 786, DE P 3 306 299.4.

Further studies on this molecule have now allowed the Applicant to set up novel processes for the preparation of morniflumate, which are particularly advantageous on industrial scale.

French pat. n° 7963M discloses a preparation process of morniflumate in form of the hydrochloride, starting from niflumic acid and N-(β -chloroethyl)morpholine (reaction scheme I).

**SCHEME I**

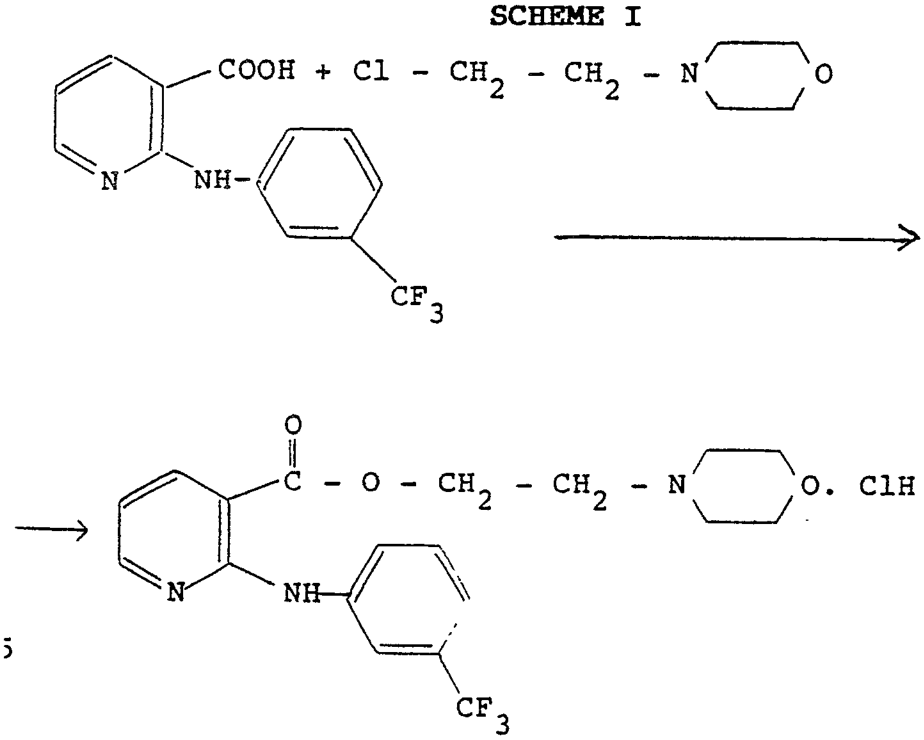

In the same patent and in the subsequent addition certificate published with FR pat. n° 2 187 317, other alternative methods for the preparation of niflumic acid esters and analogous thereof are disclosed,

which consist of the following operative steps:

a) reacting niflumic acid chloride (or an analogous) with the desired alkanolamine and recovering the final product in form of the hydrochloride (reaction scheme II):

SCHEME II

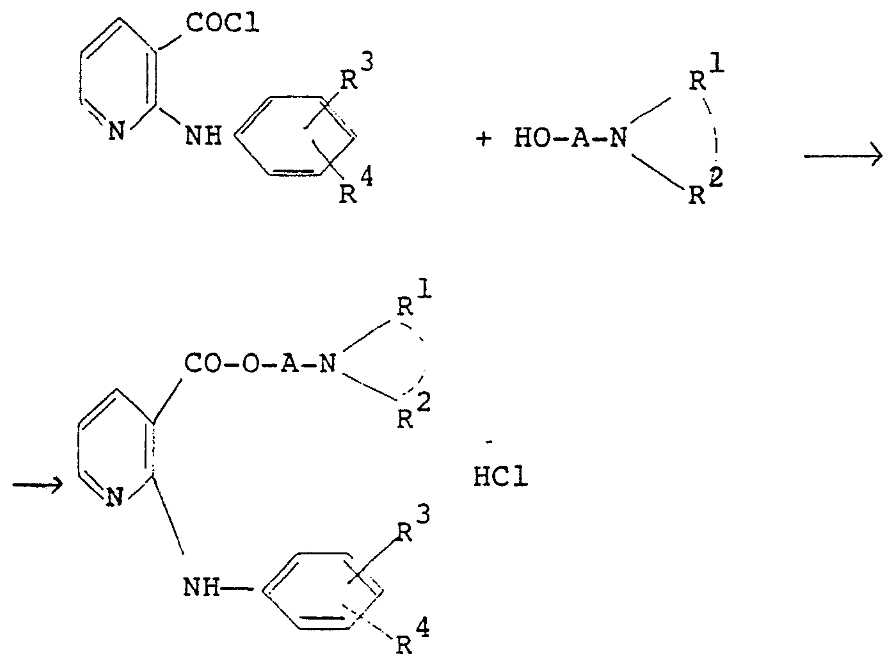

b) preparing the niflumic acid sodium salt (or an analogous compound), condensing with the desired haloalkylamine and recovering the product in form of the free base (reaction scheme III):

SCHEME III

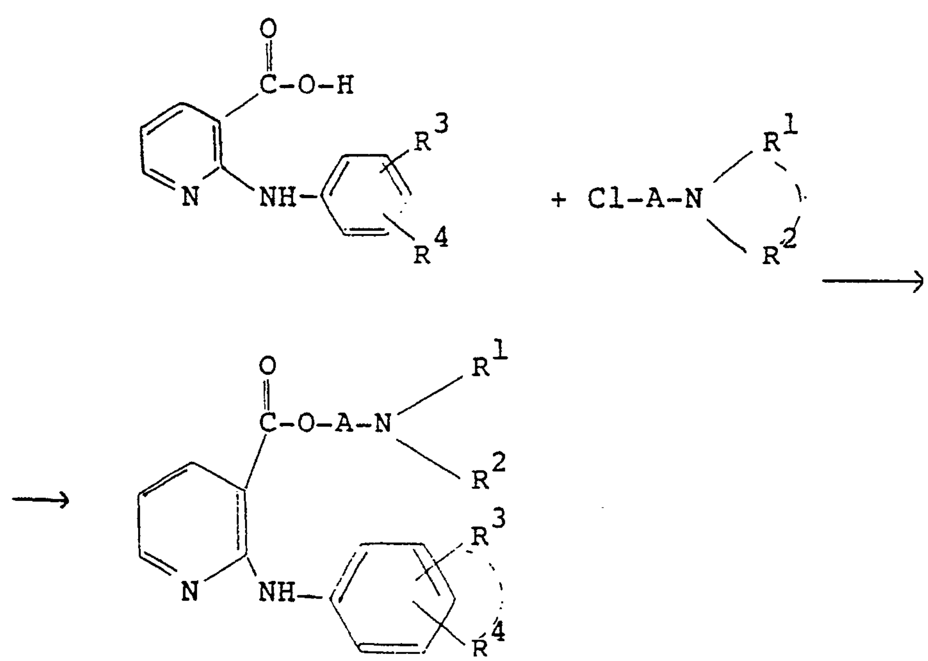

wherein:
A is a lower alkylene group;
$R_1$ e $R_2$ separately can be lower alkyl or, taken together with the nitrogen atom, can be a 5- or 6-membered heterocycle comprising another heteroatom such as O or N;

$R_3$ and $R_4$, which can be the same or different, are hydrogen, halogen, lower alkyl, lower alkoxy, lower haloalkyl;

M is an alkali metal.

As the final result from the preparation processes of schemes II and III, an hydrochloride is obtained, as in the reaction medium hydrochloric acid forms deriving, in the first case, from the esterification of niflumic acid with a chloroalkylamine and, in the second one, from the reaction of niflumic acid chloride with the desired hydroxyalkylamine.

The isolation of morniflumate (or of an anologous ester) as the free base for use in pharmaceutical formulations requires therefore a further step involving a decrease in the final product yields. Moreover, the processes for the preparation of morniflumate according to the reaction schemes I, II and III envisage the use of niflumic acid as the starting product.

On its turn, niflumic acid is obtained according to Hoffmann C. and Faure A. in Bull. Soc. Chim. France 7, 2316-2319, 1966, by reacting 3-trifluoromethylaniline with 2-chloronicotinic acid, in a yield stated to be 87% by mole.

French pat. n° 7963M states that 0,046 mole of morniflumate hydrochloride, in a 37% (by mole) yield, are obtained by esterification of 0,124 mole of niflumic acid with a slight excess (0,15 mole) of N-(β - chloroethyl)morpholine in isopropanol under reflux, for 8 hours. Therefore, the total yield in morniflumate hydrochloride, starting from 2-chloronicotinic acid, can be evaluated to about 32% by mole. The subsequent neutralization step of the compound, in order to separate the free base, further reduces the final yield of the process.

The alternative preparation route illustrated in reaction scheme III, (and described in addition certificate FR 2 187 317), which is applied to morniflumate analogous, even though allows to recover the final product as the free base, makes use of an alkali salt of the starting acid, thus involving a further step for the preparation and recovery of the salt.

The present invention provides novel industrially applicable processes for the preparation of morniflumate, or the analogous thereof, in form of the free bases, by means of a simplified procedure and in remarkably higher yields than those of the prior art processes.

The first process, in only two steps, gives the desired product as the free base (5) by preparing a 2-chloronicotinic acid aminoalkyl ester (3) and subsequently condensing said ester with an aniline derivative (4), according to the following reaction scheme IV:

SCHEME IV

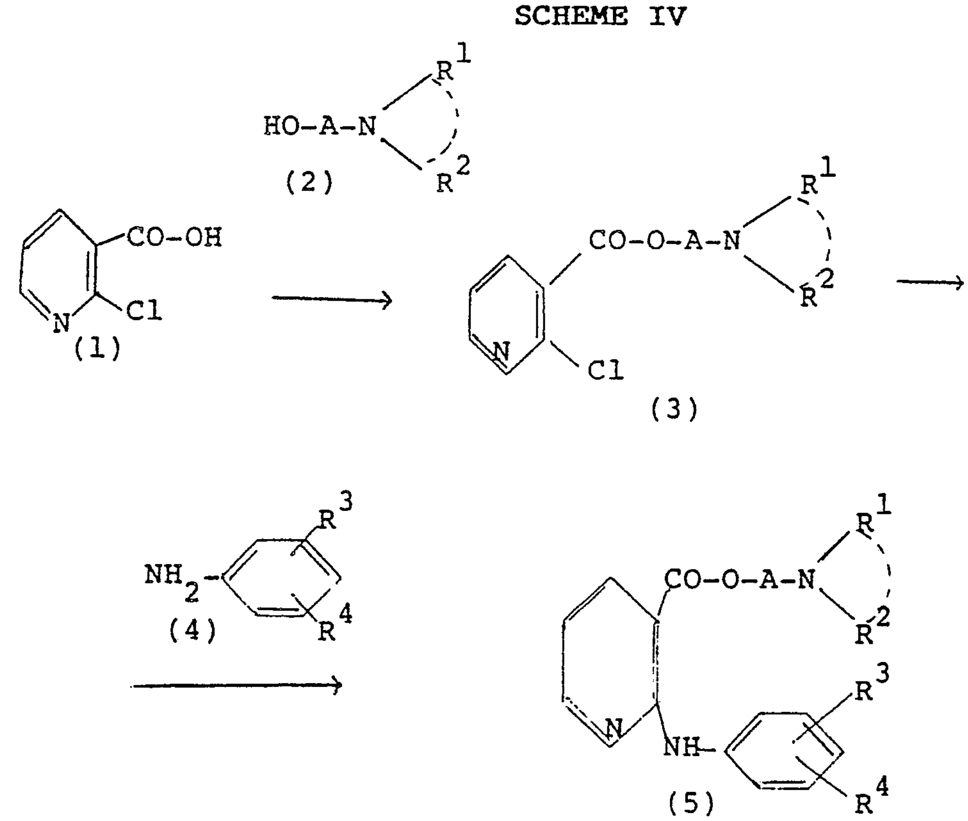

(2)
(1)
(3)
(4)
(5)

wherein A, $R_1$, $R_2$, $R_3$ and $R_4$ have the same meanings as defined in reaction schemes II and III.

According to this first process, 2-chloronicotinic acid (1), in form of the acid chloride, is reacted with an at least equivalent (by mole) amount of an alkanolamine (2) in the presence of an organic base, preferably a tertiary amine such as triethylamine, in a sufficient amount to neutralize the formed hydrochloric acid. The reaction is carried out in an inert solvent and at a temperature from 20 to 80 °C.

The resulting 2-chloronicotinic acid aminoalkyl ester (3) is reacted with an aniline derivative (4) at a temperature from 100 to 150 °C, in the presence of an inorganic base, consisting of a metal oxide.

When the reaction is over, the mixture is left to cool, then the final product is recovered and purified by filtration, column chromatography and subsequent recrystallization.

According to the second process, 2-chloronicotinic acid methyl ester (6) is prepared and subsequently condensed with an aniline derivative (4) to obtain niflumic acid methyl ester or an analogous thereof (7), which is finally transesterified with an appropriate alkanolamine (2) to give the desired product as the free base (5). This process is particularly advantageous due to the high final yield, which is more than 50% by mole. The process is illustrated in the following reaction scheme V:

**SCHEME V**

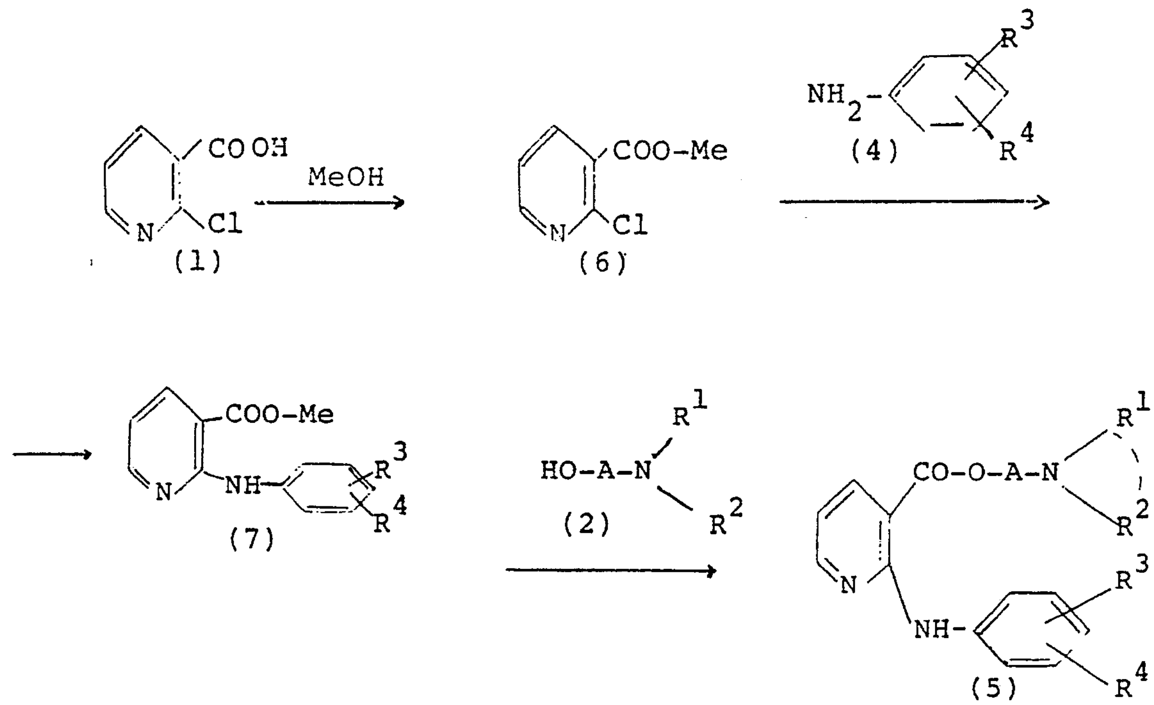


(1) (6) (4) (7) (2) (5)

2-Chloronicotinic acid, in form of the acid chloride, is treated with at least an equivalent (by mole) amount of methanol, in an organic inert solvent, in the presence of an appropriate base, preferably triethylamine, in a sufficient amount to neutralize the hydrochloric acid formed during the reaction. Then the so obtained methyl 2-chloronicotinate (6) is condensed with an aniline derivative (4), carrying out the reaction under similar conditions to those above described for the reaction of 2-chloronicotinic acid aminoalkyl ester (3) with a corresponding aniline derivative (4).

The final transesterification reaction of niflumic acid methyl ester or of an analogous thereof (7) with an at least equivalent (by mole) amount of a suited alkanolamine (2) is carried out at a temperature from 100 to 150 °C for 1-3 hours in an inert solvent, in the presence of a catalyst, slowly distilling the methanol which forms during the reaction.

When the reaction is over, the mixture is left to cool, then the final product is recovered by filtration and subsequently recrystallized.

In the following examples, which illustrate the invention in more detail, the used compounds correspond to the compounds of general formula 2, 3, 4, 5 and 7, in which:

A = $-(CH_2)_2-$;

$R_1$ and $R_2$, together with the nitrogen atom, form a morpholino ring;

$R_3$ = 3-$CF_3$, and

$R_4$ = H

**EXAMPLE 1: Preparation of 2-chloronicotinic acid 1(2-morpholinoethyl)ester (3)**

409,5 g of thionyl chloride are slowly added to 100 g of 2-chloronicotinic acid (1), under stirring, and the resulting suspension is stirred for about 5 minutes. The reaction mixture is heated and kept to ebollition for 60 minutes, to obtain a clear solution which is evaporated under vacuum. The residue is taken up into 700 ml of anhydrous toluene and evaporated again under vacuum to a 250 ml final volume. The obtained solution is dropped, during 30 minutes, into a solution of 91,5 g of 2-morpholinoethanol (2) and 73 g of triethylamine in 500 ml of anhydrous toluene, always keeping temperature at about 20 °C. The mixture is stirred for 2 hours, then cooled to +5 °C and the precipitated triethylamine hydrochloride is filtered and washed with 50 ml of toluene. The organic solution is washed with 250 ml of 5% p/v aqueous sodium carbonate, then with 2 x 200 ml of water. The solution is dried over sodium sulfate, filtered and evaporated under vacuum to completely remove the solvent. 154 g of a straw-colored oil are obtained, consisting of 2-chloronicotinic acid (2-morpholinoethyl) ester, having boiling point 250 °C, the structure of which is confirmed by IR and NMR spectra. Yield about 90% by mole.

**EXAMPLE 2: Preparation of morniflumate base (5)**

50 g of 2-chloronicotinic acid (2-morpholinoethyl) ester, obtained as described in Example 1, are mixed in 300 ml of xylene with 33 g of 3-trifluoromethylaniline (4), 15 g of zinc oxide and 0,25 g of iodine. The mixture is heated to 110 °C for 50 hours, 6 g more of 3-trifluoromethylaniline being added after 30 hours. The mixture is cooled, added with 300 ml of methylene chloride, stirred for 10 minutes; the inorganic phase is filtered and washed with 50 ml of methylene chloride. The solution is concentrated under vacuum to obtain a viscous oil which is purified by chromatography on silica gel column. The eluate is evaporated to dryness under vacuum and the residue is crystallized from 80 ml of isopropanol. The resulting crystalline product is filtered and dried under vacuum at 40 °C.

25,6 g of morniflumate base are obtained, having a melting point of 74-78 °C and a structure which is confirmed by IR and NMR spectra. Yield about 35% by mole.

**EXAMPLE 3: Preparation of methyl 2-chloronicotinate (6)**

81,9 g of thionyl chloride are slowly added to 20 g of 2-chloronicotinic acid (1), under stirring, and the suspension is stirred for 5 minutes. The mixture is heated to reflux for 60 minutes till obtaining a clear solution which is evaporated under vacuum. The residue is taken up into 200 ml of anhydrous toluene and concentrated under vacuum to 100 ml. The obtained solution is slowly dropped into a solution of 9 g of methanol and 14,6 g of triethylamine in 100 ml of anhydrous toluene, keeping temperature at about 20 °C, under stirring. The mixture is left to stand for 5 hours, then cooled to +5 °C and the precipitated triethylamine hydrochloride is filtered and washed with 20 ml of toluene. The organic phase is washed with 50 ml of 5% p/v aqueous sodium hydrogen carbonate, then with 2 x 200 ml of water, dried over sodium sulfate, filtered and concentrated under vacuum to remove the solvent.

21,35 g of methyl 2-chloronicotinate are obtained in form of a slightly yellow oil, having b.p. 225-230 °C, the structure of which is confirmed by the IR and NMR spectra. Yield about 98% by mole.

**EXAMPLE 4: Preparation of methyl morniflumate (7)**

20 g of methyl 2-chloronicotinate (6), obtained as described in Example 3, are mixed in 70 ml of xylene with 20,7 g of m-trifluoromethylaniline (4), 9,5 g of zinc and 115 mg of iodine. The mixture is heated to 110 °C for 25 hours, then cooled, added with 200 ml of ethyl acetate, stirred for 5 minutes and filtered; the inorganic phase is washed with 70 ml of ethyl acetate. The mixture is concentrated under vacuum to about 70 ml, filtered and washed with 20 ml of ethyl acetate, then evaporated to dryness under reduced pressure. The resulting residue is purified by chromatography on a silica gel column. The eluate is evaporated to dryness under vacuum and the residue is taken up into 30 ml of boiling methanol. The solution is cooled, filtered, dried in oven under vacuum at 40 °C, to obtain 22,3 g of methyl morniflumate having a m.p. of 71,5-72,1 °C, and a structure which is confirmed by the IR and NMR spectra. Yield about 65% by mole.

**EXAMPLE 5: Preparation of morniflumate base (5)**

15 g of methyl morniflumate (7) prepared as described in Example 4 are mixed with 10 g of 2-morpholinoethanol (2) in 200 ml of anhydrous toluene. 0,04 g of sodium metal are added and the mixture is heated and slowly distilled for about 3 hours, then evaporate under vacuum, cooled, taken up into 200 ml of methylene chloride and washed with 3 x 80 ml of water. The organic phase is dried over sodium sulfate, filtered and evaporated to dryness under vacuum. The residue is taken up into 35 ml of boiling isopropanol, cooled to 4° C, filtered and dried at 40° C under vacuum. 16 g of morniflumate base are obtained, having a m.p. of 74-78° C, the structure of which is confirmed by the IR and NMR spectra. Yield about 80% by mole.

## Claims

1. A process for the preparation of the compounds of general formula:

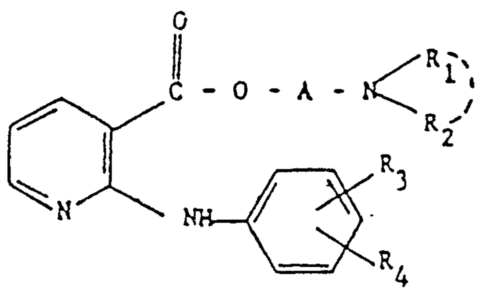

(5)

wherein:
A is a lower alkylene group;
$R_1$ and $R_2$, separately, are a lower alkyl or, taken together with the nitrogen atom, form a 5- or 6-membered heterocycle which can contain another heteroatom O or N;
$R_3$ and $R_4$ which can be the same or different, are hydrogen, halogen, lower alkyl, lower alkoxy, lower haloalkyl,
which process comprises the following steps:

a) esterificating 2-chloronicotinic acid with an alkanolamine of formula:

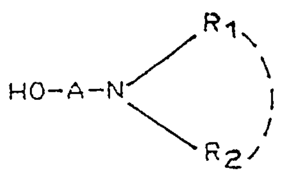

(2)

wherein A, $R_1$ and $R_2$ have the above mentioned meanings;

b) condensing the obtained ester with an aniline derivative of formula:

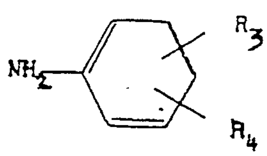

(4)

in which $R_3$ and $R_4$ have the above mentioned meanings.

2. A process according to claim 1, in which:

a) 2-chloronicotinic acid (1), in the acid chloride form, is reacted with an at least equivalent (by mole) amount of the desired alkanolamine (2) in an inert solvent and in the presence of a tertiary amine and at a temperature of 20 to 80° C;

b) 2-chloronicotinic acid aminoalkyl ester (3) and the aniline derivative (4) are reacted in a 1:2 molar ratio, in an inert solvent, in the presence of stoichiometric amounts of an heavy metal oxide and of iodine in

catalytic amounts, at a temperature from 100 to 150 °C for 30-50 hours.

3. A process according to claim 2, in which the tertiary amine is triethylamine.

4. A process according to claim 2, in which the heavy metal oxide is zinc oxide.

5. A process for the preparation of the compounds of general formula (5), which process comprises the following steps:

a) preparing 2-chloronicotinic acid methyl ester;

b) condensing methyl 2-chloronicotinate with an aniline derivative of general formula:

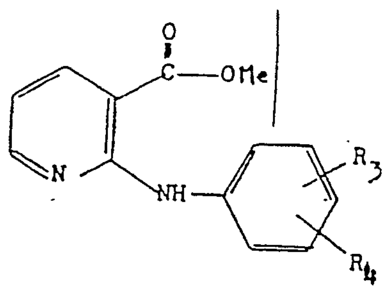

(7)

wherein $R_3$ and $R_4$ have the meanings defined in claim 1;

c) transesterificating the methyl ester (7) with an alkanolamine of formula:

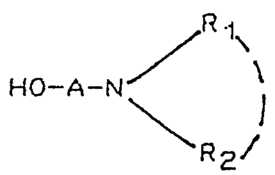

(2)

wherein A, $R_1$ and $R_2$ have the meanings described in claim 1.

6. A process according to claim 5, which process comprises the following steps:

a) reacting 2-chloronicotinic acid (1), in form of the acid chloride, with a methanol excess in an anhydrous medium, in the presence of an inert solvent and of a tertiary amine, at a temperature of about 20 °C for 4-6 hours, to obtain 2-chloronicotinic acid methyl ester (6);

b) condensing the so obtained ester with the desired aniline derivative (4) with the same process as described in claim 2, step b), to obtain the methyl ester of niflumic acid or of an analogous thereof (7);

c) subsequently transesterificating said methyl ester with an at least equivalent (by mole) amount of the desired alkanolamine (2) in an inert anhydrous solvent, in the presence of catalytic amounts of an alkali metal, at a temperature of 100-150 °C for 2-4 hours, distilling continuously the methanol which forms in the reaction medium.

7. A process for the preparation of a compound of general formula (5) according to claims 1, 2, 4 and 5, in which A is a $-(CH_2)_2-$ group, $R_1$ and $R_2$, together with the nitrogen atom , form a morpholino ring, $R_3$ is 3-trifluoromethyl and $R_4$ is hydrogen.

8. The 2-chloronicotinic acid 1-(2-morpholinoethyl) ester of formula

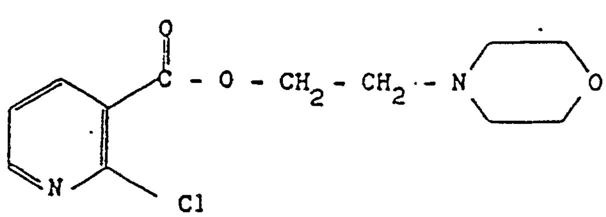

as an intermediate for the preparation of a compound according to claims 1, 2 and 7.

9. Pharmaceutical compositions for the oral administration, containing as the active ingredient morniflumate or analogous thereof, prepared according to the processes of claims 1-7, in amounts from 0,3 to 0,8 g per unitary dose.

10. Morniflumate and analogous thereof, prepared according to claims 1-9 for use in therapy for the

treatment of inflammatory affections and painful and febrile conditions.

Claims for the following Contracting States: ES, GR.

1. A process for the preparation of the compounds of general formula:

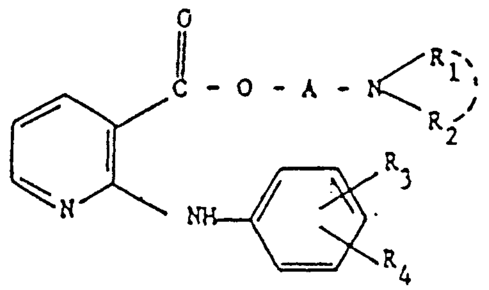

(5)

wherein:
A is a lower alkylene group;
$R_1$ and $R_2$, separately, are a lower alkyl or, taken together with the nitrogen atom, form a 5- or 6-membered heterocycle which can contain another heteroatom O or N;
$R_3$ and $R_4$, which can be the same or different, are hydrogen, halogen, lower alkyl, lower alkoxy, lower haloalkyl,
which process comprises the following steps:

a) esterificating 2-chloronicotinic acid with an alkanolamine of formula:

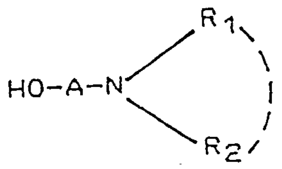

(2)

wherein A, $R_1$ and $R_2$ have the above mentioned meanings;

b) condensing the obtained ester with an aniline derivative of formula:

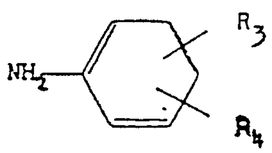

(4)

in which $R_3$ and $R_4$ have the above mentioned meanings.

2. A process according to claim 1, in which:

a) 2-chloronicotinic acid (1), in the acid chloride form, is reacted with an at least equivalent (by mole) amount of the desired alkanolamine (2) in an inert solvent and in the presence of a tertiary amine and at a temperature of 20 to 80 °C;

b) 2-chloronicotinic acid aminoalkyl ester (3) and the aniline derivative (4) are reacted in a 1:2 molar ration, in an inert solvent, in the presence of stoichiometric amounts of an heavy metal oxide and of iodine in catalytic amounts, at a temperature from 100 to 150 °C for 30-50 hours.

3. A process according to claim 2, in which the tertiary amine is triethylamine.

4. A process according to claim 2, in which the heavy metal oxide is zinc oxide.

5. A process for the preparation of the compounds of general formula (5), which process comprises the following steps:

a) preparing 2-chloronicotinic acid methyl ester;

b) condensing methyl 2-chloronicotinate with an aniline derivative of general formula:

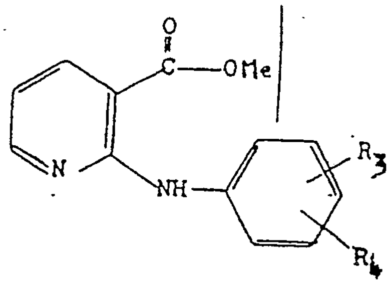

(7)

wherein $R_3$ and $R_4$ have the meanings defined in claim 1;

c) transesterificating the methyl ester (7) with an alkanolamine of formula:

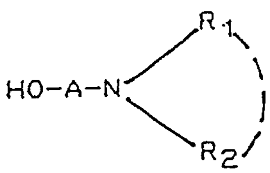

(2)

wherein A, $R_1$ and $R_2$ have the meanings described in claim 1.

6. A process according to claim 5, which process comprises the following steps:

a) reacting 2-chloronicotinic acid (1), in form of the acid chloride, with a methanol excess in an anhydrous medium, in the presence of an inert solvent and of a tertiary amine, at a temperature of about 20 °C for 4-6 hours, to obtain 2-chloronicotinic acid methyl ester (6);

b) condensing the so obtained ester with the desired aniline derivative (4) with the same process as described in claim 2, step b), to obtain the methyl ester of niflumic acid or of an analogous thereof (7);

c) subsequently transesterificating said methyl ester with an at least equivalent (by mole) amount of the desired alkanolamine (2) in an inert anhydrous solvent, in the presence of catalytic amounts of an alkali metal, at a temperature of 100-150 °C for 2-4 hours, distilling continuously the methanol which forms in the reaction medium.

7. A process for the preparation of a compound of general formula (5) according to claims 1, 2, 4 and 5, in which A is a $-(CH_2)_2-$ group, $R_1$ and $R_2$, together with the nitrogen atom , form a morpholino ring, $R_3$ is 3-trifluoromethyl and $R_4$ is hydrogen.